# EUROPEAN PATENT APPLICATION

(11) **EP 3 266 372 A1**
(43) Date of publication of application: **10.01.2018**
(21) Application number: 16183637.4
(22) Date of filing: 10.08.2016
(51) Int. Cl.: A61B 5/024, A61B 5/11, A61B 5/00

(54) **METHOD AND DEVICE FOR REAL-TIME MONITORING OF MAXIMAL OXYGEN CONSUMPTION**

(30) Priority: 08.03.2016 US 201615064587
(71) Applicant: bOMDIC Inc., Taipei City 104 (TW)
(72) Inventor: Cheng, Shih-Heng, 104 Taipei (TW)
(74) Representative: CMS Cameron McKenna Nabarro Olswang LLP

(57) **Abstract**

The present disclosure provides an exercise monitoring device. The exercise monitoring device comprises a sensor module, a processing module, a storage module, and a user interface. The present disclosure also provides a method for estimating maximal oxygen consumption and/or future total exercise time by obtaining a person's physiological data and exercise data. The present disclosure further provides a method for calibrating the future total exercise time by environmental conditions to form an environmental specific total exercise time.

## Description

### FIELD OF THE INVENTION

The present invention relates generally to an exercise monitoring method and device. More specifically, the present invention relates to an exercise monitoring method and device utilizing stamina of a user for monitoring a real-time maximal oxygen consumption of a user and/or predicting a result of future exercise of the user.

### BACKGROUND OF THE INVENTION

Doing exercise has become more and more popular in modern societies. Other than professional athletes, many individuals not only doing exercise for health considerations, but also seek to know how well do they perform during the exercise. Traditionally, this could only be achieved accurately by having fitness tests in a laboratory with various testing equipment. Alternatively, individuals can hire personal fitness coach to assist themselves understanding their own performance.

Recently, various types of exercise performance evaluation have been rapidly developed and applied by professional athletes, sports enthusiasts, fitness coach, or even individuals. Among all sorts of exercise performance evaluation, maximal oxygen consumption is widely used and proven to be effective. However, the maximal oxygen consumption of any individuals cannot be easily assessed without spending an hour on a treadmill in a laboratory with a mouthpiece which receives the individuals' breathing in order to monitor and analyze changes in breathing rate, oxygen consumption, carbon dioxide production, etc. Therefore, it is not convenient to obtain an individual's exercise performance while doing daily exercise practices or training.

In view of the above, what is needed is an exercise monitoring device and method which reliably indicates a maximal oxygen consumption of any user while doing exercise anywhere under a real-time basis without wearing a mouthpiece in the laboratory.

### SUMMARY OF THE INVENTION

The present invention provides a maximal oxygen consumption estimation method which comprises the following steps: receiving a physiological data from a first sensor; estimating a rate of stamina consumption base on the physiological data by a processing module; calculating an all-out exercise time base on the rate of stamina consumption by the processing module; estimating an exercise capability base on the all-out exercise time by the processing module; receiving an exercise data from a second sensor; calculating a kinetic energy exertion base on the exercise data by the processing module; estimating an average oxygen consumption base on the kinetic energy exertion by the processing module; estimating a maximal oxygen consumption base on the average oxygen consumption and the exercise capability by the processing module; sending the maximal oxygen consumption to a user interface; displaying the maximal oxygen consumption by the user interface.

The present invention further provides a future total exercise time estimation method which comprises the following steps: receiving a physiological data from a first sensor; estimating a rate of stamina consumption base on the physiological data by a processing module; calculating an all-out exercise time base on the rate of stamina consumption by the processing module; estimating an exercise capability base on the all-out exercise time by the processing module; receiving an exercise data from a second sensor; calculating a kinetic energy exertion base on the exercise data by the processing module; estimating an average oxygen consumption base on the kinetic energy exertion by the processing module; estimating a maximal oxygen consumption base on the average oxygen consumption and the exercise capability by the processing module; estimating a future total exercise time base on the maximal oxygen consumption and a default displacement; sending the future total exercise time to a user interface; displaying the future total exercise time by the user interface.

The present invention further provides a future total exercise time estimation method which comprises the following steps: receiving a historical exercise model from a terminal device, wherein the historical exercise model comprises a plurality of heart rate and a plurality of displacement corresponding to the heart rate; calculating a plurality of heart rate percentage based on the plurality of heart rate; calculating a plurality of speed based on the plurality of displacement; estimating a maximal oxygen consumption based on the plurality of heart rate percentage and the plurality of speed, wherein the maximal oxygen consumption is negative correlated to the plurality of heart rate percentage and positive correlated to the plurality of speed; estimating a future total exercise time based on a default displacement and the maximal oxygen consumption, wherein the future total exercise time is positive correlated to the default displacement, and wherein the future total exercise time is negative correlated to the maximal oxygen consumption; generating a data array comprising the maximal oxygen consumption and the future total exercise time; sending the data array to the terminal device.

### BRIEF DESCRIPTION OF THE DRAWINGS

The accompanying drawings illustrate one or more embodiments of the invention and together with the written description, serve to explain the principles of the invention. Wherever possible, the same reference numbers are used throughout the drawings to refer to the same or like elements of an embodiment, and wherein:
Fig. 1 is a schematic block diagram of an exercise monitoring device according to at least one embodiment of the present invention.
Fig. 2 is schematic illustrations of the relationship between lactic acid concentration and heart rate of a user according to at least one embodiment of the present invention.
Fig. 3 is a schematic illustration of changes of lactic acid concentration and stamina level on the same time scale of a user during exercise according to at least one embodiment of the present invention.
Fig. 4 is a schematic illustration of relationship between stamina level and all-out exercise time of a user during exercise according to at least one embodiment of the present invention.
Fig. 5A is a schematic illustration of relationship between exercise capability and all-out exercise time of a user according to at least one embodiment of the present invention.
Fig. 5B is a schematic illustration of compositions affecting the relationship between the exercise capability and all-out exercise time in Fig. 5A according to at least one embodiment of the present invention.
Fig. 6 is a flowchart of a method for estimating maximal oxygen consumption according to at least one embodiment of the present invention.
Fig. 7 is a flowchart of a method for estimating future exercise time according to at least one embodiment of the present invention.
Fig. 8 is a schematic illustration of the mapping concept between stamina level and RPE according to at least one embodiment of the present invention.
Fig. 9 is a flowchart of a method for estimating maximal oxygen consumption according to at least one embodiment of the present invention.
Fig. 10 is a flowchart of a method for estimating future exercise time according to another embodiment of the present invention.
Fig. 11 is a schematic block diagram of the exercise monitoring device with a heart rate sensor and a GPS according to at least one embodiment of the present invention.
Fig. 12 is a schematic block diagram of the exercise monitoring device with an external sensor module and an external user interface according to at least one embodiment of the present invention.
Fig. 13 is a schematic example of the data array generated in Fig. 10.

In accordance with common practice, the various described features are not drawn to scale and are drawn to emphasize features relevant to the present disclosure. Like reference characters denote like elements throughout the figures and text.

### DETAILED DESCRIPTION OF THE INVENTION

The present invention will now be described more fully hereinafter with reference to the accompanying drawings, in which exemplary embodiments of the invention are shown. This invention can, however, be embodied in many different forms and should not be construed as limited to the embodiments set forth herein. Rather, these embodiments are provided so that this disclosure will be thorough and complete, and will fully convey the scope of the invention to those skilled in the art. Like reference numerals refer to like elements throughout.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," or "includes" and/or "including" or "has" and/or "having" when used herein, specify the presence of stated features, regions, integers, steps, operations, elements, and/or components, but do not preclude the presence or addition of one or more other features, regions, integers, steps, operations, elements, components, and/or groups thereof.

It will be understood that the term "and/or" includes any and all combinations of one or more of the associated listed items. It will also be understood that, although the terms first, second, third etc. can be used herein to describe various elements, components, regions, parts and/or sections, these elements, components, regions, parts and/or sections should not be limited by these terms. These terms are only used to distinguish one element, component, region, part or section from another element, component, region, layer or section. Thus, a first element, component, region, part or section discussed below could be termed a second element, component, region, layer or section without departing from the teachings of the present invention.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

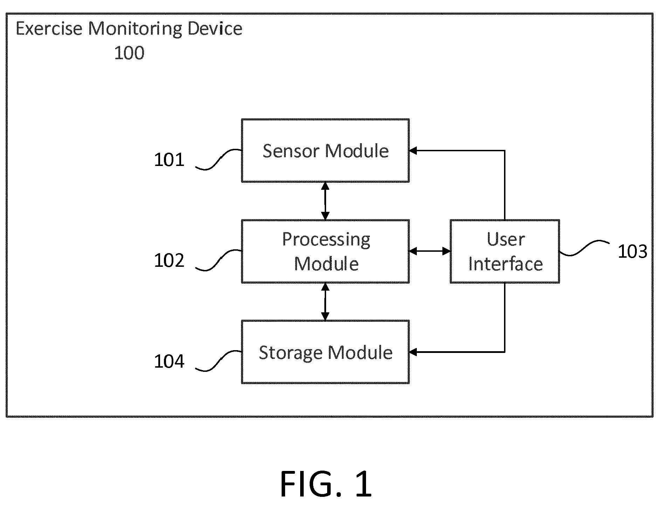

The description will be made as to the embodiments of the present invention in conjunction with the accompanying drawings in Figs. 1~13. Reference will be made to the drawing figures to describe the present invention in detail, wherein depicted elements are not necessarily shown to scale and wherein like or similar elements are designated by same or similar reference numeral through the several views and same or similar terminology.

Fig. 1 is a schematic block diagram of an exercise monitoring device according to at least one embodiment of the present invention.

The exercise monitoring device 100 comprises a sensor module 101, a processing module 102, a user interface 103, and a storage module 104.

In one embodiment of the present invention, the sensor module 101 can comprise at least one physiological sensor for sensing and measuring physiological signals of a user. For example, the physiological signal comprises at least one of the following: EKG signal, pulse, heart rate, breathing pattern, glycogen concentration, oxygen concentration (SpO2) from pulse oximeter, oxygen concentration (StO2) from tissue oximeter, and oxygen concentration measured from front lobe of a person. The tissue oximeter can be Near Infra-Red Spectroscopy tissue oximeter, etc. The oxygen concentration at front lobe is correlated to RPE of a person.

In another embodiment of the present invention, the sensor module 101 can comprise a plurality of sensors for sensing and measuring both physiological signals of a user like mentioned before and non-physiological signals. In terms of non-physiological signals, the sensor module 101 can comprise various types of non-physiological sensors such as pedometer, speedometer, accelerometer, gyroscope, G-sensor, etc.

In at least one embodiment of the present invention, the processing module 102 is a hardware such as a processor, a microcontroller or a microprocessor with auxiliary circuits that carries out instructions of a computer program by performing the basic arithmetical, logical, and input/output operations of the exercise monitoring device. Many different products on the market can be used as the processing module 102 such as for example but not limited to nRF52832 from Nordic Semiconductor, STM32L476 from STMicroelectronics.

In one embodiment of the present invention, the user interface 103 comprises at least one output unit (not shown) and/or at least one input unit (not shown), or any combination thereof. The output can be a display, a vibrating component or a speaker, or any combination thereof for stating the user's physiological status during the exercise or after the exercise, wherein the physiological status can comprise at least one of the following: measurement of physiological signal, stamina level, kinetic energy consumption, maximal oxygen consumption, etc. The input can be any human-machine interface such as a touch-panel, a voice receiver or a button that is capable of receiving biological information from the user, such as height, weight, age, gender and so forth. In addition, the user interface 103 can be adapted to send information directly to the sensor module 101, the processing module 102 or the storage module 104. The inputted information can be processed by the processing module 102 and sent to the output for the user to know the user's current body condition. For example, BMI value, etc.

In one embodiment of the present invention, the storage module 104 can be any type of volatile or non-volatile memory for storing instructions of a computer program to be carried out by the processing module 102, biological information inputted by the user using the user interface 103, and exercise information from the sensor module 101 and/or the processing module 102.

It should be noticed that the term, stamina, refers to the ability of a user to exert himself/herself and remain active for a period of time. The less the stamina of a person, the less time the person can continue exercising providing the same exercise intensity without rest.

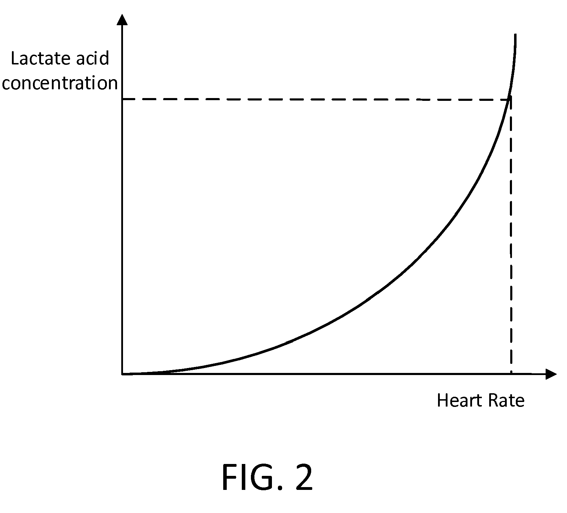

Fig. 2 is schematic illustrations of the relationship between lactic acid concentration and heart rate of a user according to at least one embodiment of the present invention.

Referring to Fig. 2, the average heart rate of the user is positively correlated to the lactic acid concentration, which can be approximated with a linear regression model, a non-linear regression model, a piecewise function, other mathematical models or any combination thereof. Thus, the lactic acid concentration associated with the lactic acid accumulated in the blood stream is able to be estimated base on the heart rate of the user.

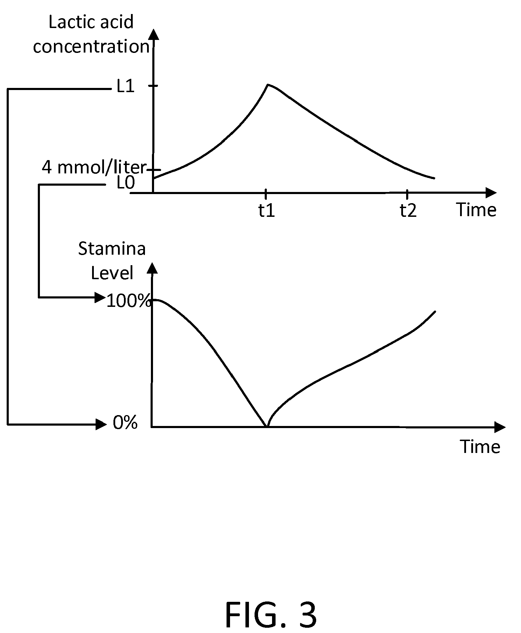

Fig. 3 is a schematic illustration of changes of lactic acid concentration and stamina level on the same time scale of a user during exercise according to at least one embodiment of the present invention.

It is well known in the art that a lactic acid concentration of 4 mmol per liter is considered as a threshold between aerobic exercise and anaerobic exercise. With aerobic exercise, oxygen is carried through the user's breath to the muscles giving muscles the energy needed to sustain the effort. With anaerobic exercise, the exercise intensity is high enough to trigger lactic acid formation, which causes discomfort and fatigue at sustained levels.

Referring to Fig. 3, the stamina level of a user is set to 100% when the lactic acid concentration is at a range of 2~6 mmol per liter. As the user continues with the exercise, the lactic acid concentration increases while the stamina level decreases. Once the lactic acid concentration reaches a threshold, i.e. lactic acid increases to L1 at t1 time, the user's stamina level reaches substantially 0%, the user can be suggested by the exercise monitoring device 100 to choose to decrease the exercise intensity to metabolize the accumulated lactic acid and thus the stamina level recovers from 0% to 100% during t1 to t2 time frame. It should be noticed that the recovery from 0% to 100% is not necessary, but a recovery from 0% to a certain percentage is better for a user to improve his/her performance during the exercise or competition. It should be noticed that, the time for different user to fully recovered, for example from 0% to 100% stamina level, is similar, for example, approximately 8 to 12 minutes. Therefore, the lactic acid concentration of a person is inverse correlative to the person's stamina level.

It should be noticed that although the aforementioned heart rate is disclosed for mapping and normalizing with the stamina level in Fig. 1-3, other physiological signal can also be implemented, such as EKG signal, pulse, heart rate, breathing pattern, glycogen concentration, oxygen concentration (SpO2) from pulse oximeter, oxygen concentration (StO2) from tissue oximeter, and oxygen concentration measured from front lobe of a person. The tissue oximeter can be Near Infra-Red Spectroscopy (NIRS) tissue oximeter, etc. Also, this concept is applicable to any embodiments of the present invention.

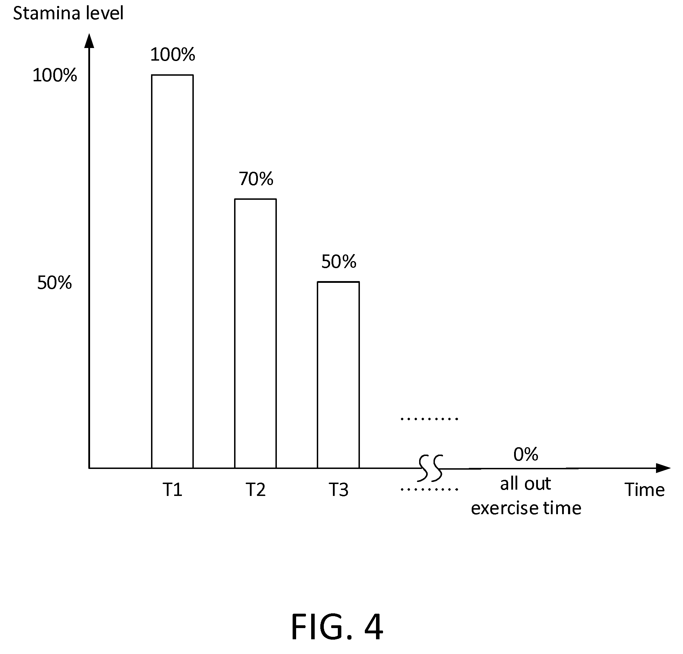

Fig. 4 is a schematic illustration of relationship between stamina level and all-out exercise time of a user during exercise according to at least one embodiment of the present invention. In Fig. 4, the stamina level is 100% at T1, 70% at T2, 50% at T3. Obviously, the stamina level will keep decrease until the user is physically not able to continue doing exercise. In other words, the stamina level should reach 0% when the user doing exercise until an all-out physical condition, wherein the all-out physical condition is given a timing "all-out exercise time". As the stamina level is constantly monitored by the exercise monitoring device 100, the all-out exercise time can be estimated accordingly. For example, the all-out exercise time in Fig. 4 can be estimated by a linear regression model, a non-linear regression model, a piecewise function, other mathematical models or any combination thereof.

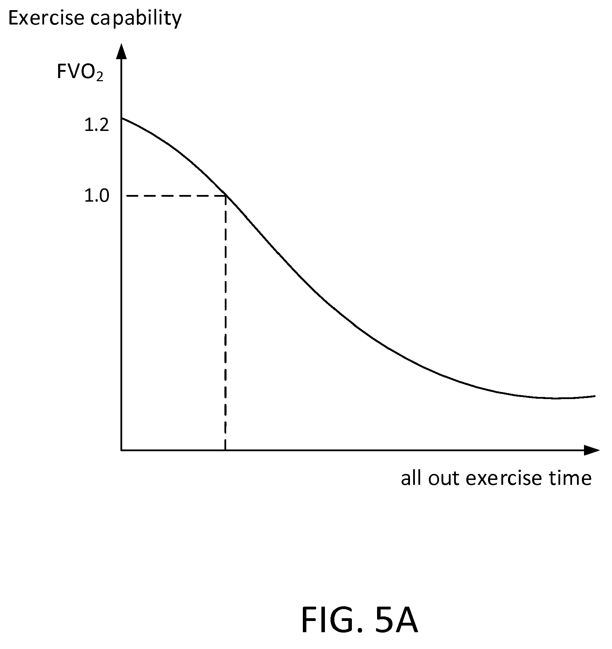

Fig. 5A is a schematic illustration of relationship between exercise capability and all-out exercise time of a user according to at least one embodiment of the present invention.

In Fig. 5A, the graph shows a user's FVO₂ drops as the all-out exercise time increases. The exercise capability which is used later on is defined as FVO₂ (Fraction of maximal oxygen consumption). In other words, the exercise capability is a user's average oxygen consumption compare to his/her maximal oxygen consumption during an all-out exercise. Alternatively, the exercise capability can also be used to describe the user's exercise efficiency. This concept should be well explained as following, i.e. it can take 15 seconds for a person to sprint a 100m, but takes 1 hour for the same person to run 5000m which is 72 seconds for every 100m in average. Therefore, the shorter the all-out exercise time, the higher the exercise efficiency is achieved. Referring back to FVO₂, the user uses very short time to exercise till all-out (100m sprinting), the FVO₂ should be relative high in comparison to a marathon (42km running). In view of the above, the longer the all-out exercise time of the user, the lower the exercise capability of the user. Thus, the exercise capability is non-linearly inverse correlative to the all-out exercise time. This is particularly useful for anyone who would like to manage his/her exercise intensity to complete a specific exercise to achieve his/her best performance, wherein exercise intensity is directly related to a person's oxygen consumption, the higher the exercise intensity, the more oxygen is taken into the person's breathe in order to meet the person's need, but the shorter the all-out exercise time is.

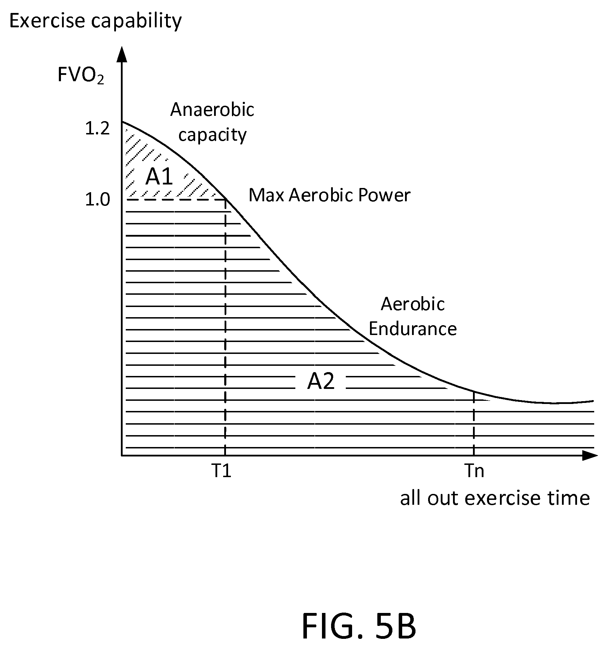

Fig. 5B is a schematic illustration of compositions affecting the relationship between the exercise capability and all-out exercise time in Fig. 5A according to at least one embodiment of the present invention.

In Fig. 5B, the graph shows three major components that will affect the curve of FVO₂ (exercise capability) over time. The three components are anaerobic capacity, max aerobic power, and aerobic endurance. The area A1 under the curve represents the anaerobic capacity, wherein the larger the area A1 is the more anaerobic energy can be exerted by a person. The area A2 under the curve represents aerobic energy, and the max aerobic power is 1.0 of FVO₂ at T1, wherein T1 is the time that a person stops using anaerobic energy. Even though, the max aerobic power is normalized to 1.0 of FVO₂ in Fig. 5B, the time T1 actually varies from person to person. Tn represents all out exercise time at any point after T1, wherein the slope (steepness) of the curve between T1 and Tn is the aerobic endurance. In other words, aerobic endurance is a person's ability to keep the FVO₂ as close as 1.0 while being active, so the steeper the curve is, the lower the aerobic endurance becomes. It should be noticed that, different people can have different anaerobic capacity, max aerobic power, and aerobic endurance because of their physical conditions such as age, gender, etc. Thus, the relationship between the exercise capability and the all-out exercise varies from person to person. For example, *FVO₂* = *f(T, MaxAerobicPower, AnaerobicCapaciy, AerobicErtdurartce),* wherein *T* is the all-out exercise time. FVO₂ = f(T, MaxAerobicPower, AnaerobicCapacity, AerobicEndurance) FVO₂ = f(T, MaxElerobicPower, AnaerobicCapacity, AerobicEndurance)In practice, an average value of anaerobic capacity, max aerobic power, and aerobic endurance can be obtained by big data analysis of athletes' performance. In addition, the average value of anaerobic capacity, max aerobic power, and aerobic endurance can be further configured using a person's biological information mentioned in Fig. 1.

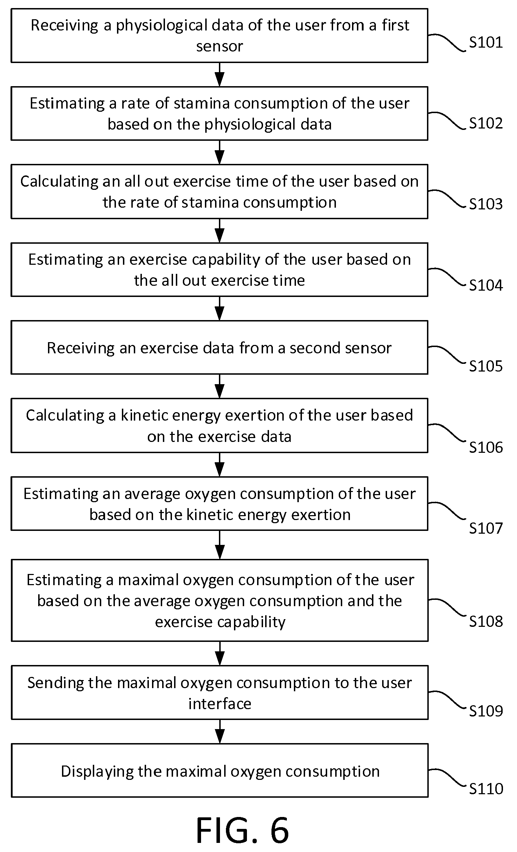

Fig. 6 is a flowchart of a method for estimating maximal oxygen consumption using the exercise monitoring device 100 in Fig. 1 according to at least one embodiment of the present invention.

Referring to Fig. 6, the following steps can be carried out to estimate maximal oxygen consumption of a user base on a physiological data of the user and an exercise data:
S101: Receiving a physiological data of the user from a physiological sensor of the sensor module 101;
S102: Estimating a rate of stamina consumption of the user base on the physiological data by the processing module 102;
S103: Calculating an all-out exercise time of the user base on the rate of stamina consumption by the processing module 102;
S104: Estimating an exercise capability of the user base on the all-out exercise time by the processing module 102;
S105: Receiving an exercise data from a non-physiological sensor of the sensor module 101;
S106: Calculating a kinetic energy exertion of the user base on the exercise data by the processing module 102;
S107: Estimating an average oxygen consumption of the user base on the kinetic energy exertion by the processing module 102;
S108: Estimating a maximal oxygen consumption of the user base on the average oxygen consumption and the exercise capability by the processing module 102
S109: Sending the maximal oxygen consumption by the processing module 102 to the user interface 103 for display.
S110: Displaying the maximal oxygen consumption by the user interface 103.

In one embodiment of the present invention, the storage module 104 can comprise a plurality of linear regression models, non-linear regression models, piecewise functions, other mathematical models or any combination thereof, corresponding to Fig. 2-5 for the processing module 102 to execute in order to perform the steps S102-S104 and S106-S107.

In one embodiment of the present invention, it should also be noticed that the steps S101-S104 can be carried out after S105-S107.

In one embodiment of the present invention, the sensor module 101 can comprise at least one physiological sensor for sensing and measuring physiological signals of a user. For example, the physiological signal comprises at least one of the following: EKG signal, pulse, heart rate, breathing pattern, glycogen concentration, oxygen concentration (SpO2) from pulse oximeter, oxygen concentration (StO2) from tissue oximeter, and oxygen concentration measured from front lobe of a person. The tissue oximeter can be Near Infra-Red Spectroscopy tissue oximeter, etc. The body composition can comprise percentages of fat, bone, water and muscle in human bodies.

In at least one embodiment of the present invention, the non-physiological sensor which can obtain the exercise data that can comprise various types of exercise parameters, such as displacement of exercise, time used for exercise, speed of running, cycling power, altitude of climbing, etc. The displacement of exercise can be a running distance, a climbing altitude, a cycling distance, etc. For example, the exercise monitoring device 100 can comprise a motion sensor, cycling power meter, pedometer, or any other speed or speed related sensors as the non-physiological sensor in the sensor module 101 to record the exercise data accordingly. Alternatively, a user can connect an external motion sensor, cycling power meter, pedometer, or any other speed or speed related sensors to the sensor module 101 of the exercise monitoring device 100 in order to record the exercise data and send to the processing module 102. The motion sensor comprises at least one of an accelerometer, a gyroscope, and a magnetometer.

In at least one embodiment of the present invention, the kinetic energy exertion is estimated by converting the exercise data such as running speed to kinetic energy in term of power (Joules). For example, *Power(t)= f(Speed(t), t).* Power(t) = f(Speed(t), t)Therefore, the kinetic energy exertion is a function of speed, wherein the kinetic energy exertion is positively correlated to the speed of exercise. Moreover, consuming oxygen gives energy, so one's average oxygen consumption (VO₂) can be estimated by kinetic energy exertion. For example, *VO₂* = *f(Power(t), t).* Namely, average oxygen consumption (VO₂) needed by the user to exert the kinetic energy is estimated in step S106 during exercise. Although speed is used in the example, other exercise parameters can be used, such as altitude of climbing, cycling power, etc.

In one embodiment of the present invention, the physiological sensor can be a combination of sensors which detects various physiological parameters, such as heart rate from optical heart rate sensor, oxygen concentration from NIRS, etc. The same concept is applicable in any embodiments of the present invention. Thus, the non-physiological sensors can also be a combination of sensors which detects various non-physiological parameters, such as acceleration from motion sensor, location from GPS, ambient temperature from thermometer, angular acceleration from gyroscope, etc.

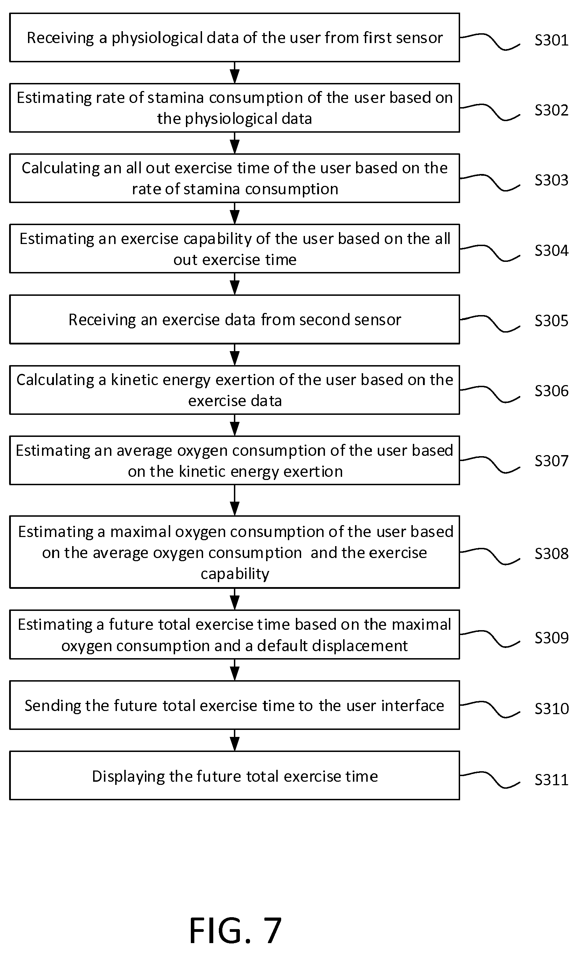

Fig. 7 is a flowchart of a method for estimating future exercise time according to at least one embodiment of the present invention.

Referring to Fig. 7, the following steps can be carried out to estimate future total exercise time of a user base on a physiological data of the user, an exercise data and a default displacement:
S301: Receiving a physiological data of the user from a physiological sensor of the sensor module 101;
S302: Estimating a rate of stamina consumption of the user base on the physiological data by the processing module 102;
S303: Calculating an all-out exercise time of the user base on the rate of stamina consumption by the processing module 102;
S304: Estimating an exercise capability of the user base on the all-out exercise time by the processing module 102;
S305: Receiving an exercise data from a non-physiological sensor of the sensor module 101;
S306: Calculating a kinetic energy exertion of the user base on the exercise data by the processing module 102
S307: Estimating an average oxygen consumption of the user base on the kinetic energy exertion by the processing module 102;
S308: Estimating a maximal oxygen consumption of the user base on the average oxygen consumption and the exercise capability by the processing module 102
S309: Estimating a future total exercise time base on the maximal oxygen consumption and a default displacement by the processing module 102;
S310: Sending the future total exercise time by the processing module 102 to the user interface 103 for display;
S311: Displaying the future total exercise time by the user interface 103.

In one embodiment of the present invention, the storage module 104 can comprise a plurality of linear regression models, non-linear regression models, piecewise functions, other mathematical models or any combination thereof, corresponding to Fig. 2-5 for the processing module 102 to execute in order to perform the steps S302-S304 and S306-S307.

It should be noticed that the steps S301-S304 can be carried out after S305-S307.

In one embodiment of the present invention, the future total exercise time is an estimation of the user's performance of an all-out exercise base on the user's maximal oxygen consumption while the user not doing the all-out exercise for real. For example, the user can complete a 5 km running with the exercise monitoring device 100 and know his/her best performance to complete a 10 km running without actually completing a 10 km running whether or not the 5 km running is completed with his/her best effort. Namely, the user can complete an exercise in ease but still being able to understand his/her performance of an all-out exercise which has not happened yet. Thus, estimation of future total exercise time is particularly useful to anyone wants to know his/her best performance without getting exhausted to complete an all-out exercise.

In one embodiment of the present invention, the default displacement can be a distance or a set of distances saved in the storage module 104 of the exercise monitoring device 100, wherein the distance or the set of distances can be chosen from 3 km, 5 km, 10 km, 21 km, 42 km, etc. Alternatively, it can be defined by the user of the exercise monitoring device 100 using the input unit of the user interface 103. It should be noticed that, the default displacement can be displacement of exercise such as a running distance, a climbing altitude, a cycling distance, etc.

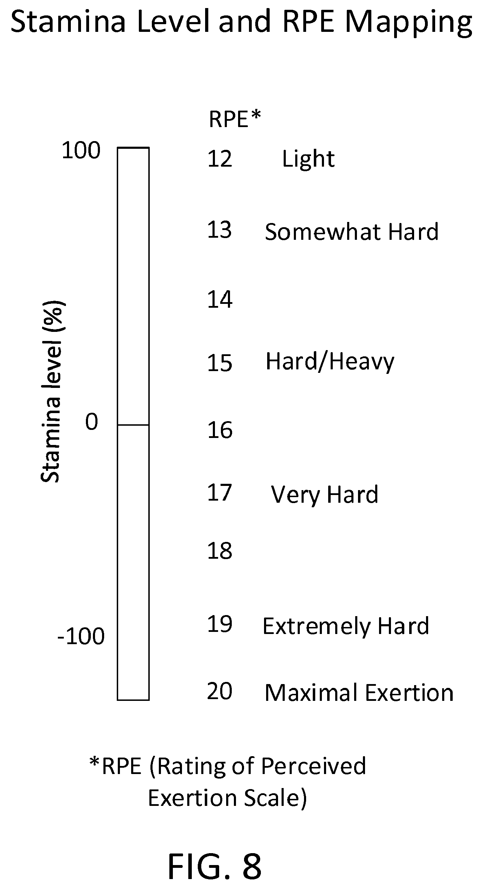

Fig. 8 is a schematic illustration of the mapping concept between stamina level and RPE according to at least one embodiment of the present invention.

Referring to Fig. 8, a mapping between the stamina level and rating of perceived exertion (RPE) is disclosed, wherein the type of RPE can be Borg Rating of Perceived Exertion Scale. The stamina level recovers when RPE value, blood lactic acid concentration (the lactic acid concentration in the blood stream) or real-time exercise loading decreases. On the other hand, the stamina level decreases when fatigue level, blood lactic acid concentration (the lactic acid concentration in the blood stream) or real-time exercise loading increases.

Continues with Fig. 8, the stamina level can be presented within a certain range, such as 100% to 0%. In addition, the stamina level is at least partially related to the RPE scale linearly or non-linearly. For example, a RPE around 12 suggests that exercise intensity is being performed at a moderate level by the user of the exercise monitoring device 100. That is to say, the user can experience "light" muscle fatigue or breathing slightly heavier than not doing any exercise, and thus a RPE around 12 can correspond to a 100% stamina level.

On the other hand, a RPE between 15 and 17 suggests that exercise intensity is being performed at a much higher level by the user of the exercise monitoring device 100. That is to say, the user can experience "hard/heavy" muscle fatigue or breathing much heavier than not doing any exercise, and thus a RPE between 15 and 17 can correspond to a 0% stamina level.

In another embodiment of the present invention, in a perspective that takes heart signal as input of the exercise monitoring device 100 as an example, the RPE scale is linearly or nonlinearly correlative to the heart rate, and thus the stamina level is also linearly or nonlinearly correlative to the heart rate. In another example, the stamina level of each user is normalized to a fixed range according to the maximum and minimum heart rate.

It should be noticed that the stamina level can be a negative value as shown in Fig. 8, wherein the stamina level being negative can trigger the exercise monitoring device 100 automatic adjusting the estimation of the stamina level according to the user's exercise intensity and thus calibrating the stamina level accordingly.

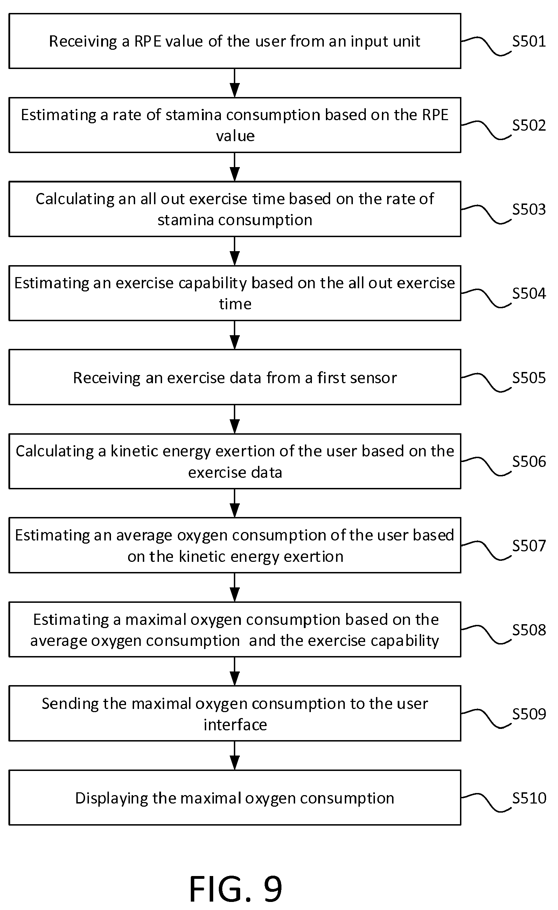

Fig. 9 is a flowchart of a method for estimating maximal oxygen consumption according to at least one embodiment of the present invention.

Referring to Fig. 9, the following steps can be carried out to estimate maximal oxygen consumption of a user base on a RPE value of the user and an exercise data:
S501: Receiving a RPE value of the user from an input unit of the user interface 103;
S502: Estimating a rate of stamina consumption base on the RPE value by the processing module 102;
S503: Calculating an all-out exercise time base on the rate of stamina consumption by the processing module 102;
S504: Estimating an exercise capability base on the all-out exercise time by the processing module 102;
S505: Receiving an exercise data from a non-physiological sensor of the sensor module 101;
S506: Calculating a kinetic energy exertion of the user base on the exercise data by the processing module 102
S507: Estimating an average oxygen consumption of the user base on the kinetic energy exertion by the processing module 102;
S508: Estimating a maximal oxygen consumption base on the average oxygen consumption and the exercise capability by the processing module 102
S509: Sending the maximal oxygen consumption by the processing module 102 to the user interface 103 for display.
S510: Displaying the maximal oxygen consumption by the user interface 103.

In one embodiment of the present invention, the storage module 104 can comprise a plurality of linear regression models, non-linear regression models, piecewise functions, other mathematical models or any combination thereof, corresponding to Fig. 2-5 for the processing module 102 to execute in order to perform the steps S502-S504 and S506-S507.

It should be noticed that the steps S501-S504 can be carried out after S505-S507.

In one embodiment of the present invention, estimation of maximal oxygen consumption in the step S108 in Fig. 6, step S308 in Fig. 7, and Step S508 in Fig. 9 can be performed by a function of the exercise capability and the average oxygen consumption, wherein the exercise capability is negatively correlated to the maximal oxygen consumption, and the average oxygen consumption is positively correlated to the maximal oxygen consumption. For example, *VO₂Max* = *f(VO₂, FVO₂)*.The same concept of estimation of maximal oxygen consumption can be applied in any other embodiments of the present invention.

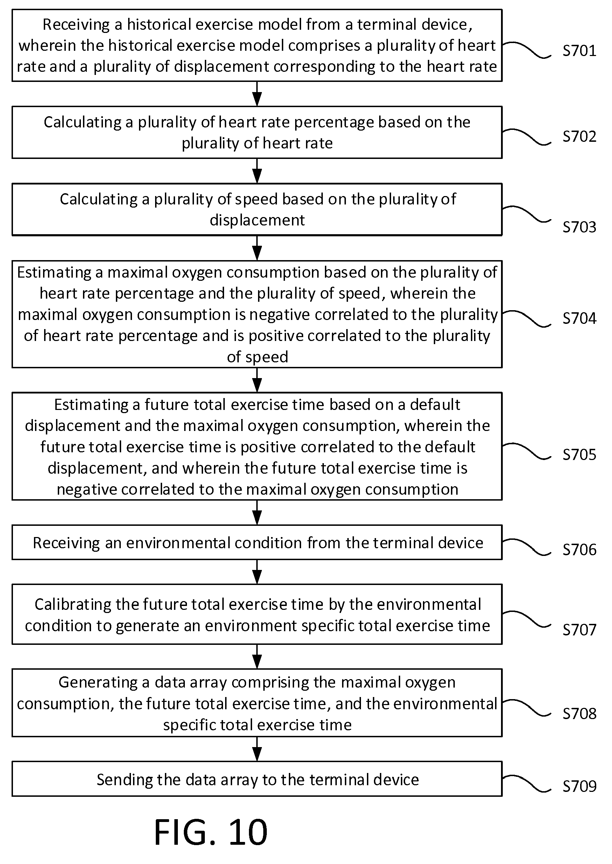

Fig. 10 is a flowchart of a method for estimating future exercise time according to another embodiment of the present invention.

Referring to Fig. 10, the following steps can be carried out to estimate a maximal oxygen consumption of a user base on a physiological data of the user and an exercise data:
S701: Receiving a historical exercise model from a terminal device, wherein the historical exercise model comprises a plurality of heart rate and a plurality of displacement corresponding to the heart rate;
S702: Calculating a plurality of heart rate percentage based on the plurality of heart rate;
S703: Calculating a plurality of speed based on the plurality of displacement;
S704: Estimating a maximal oxygen consumption based on the plurality of heart rate percentage and the plurality of speed, wherein the maximal oxygen consumption is negative correlated to the plurality of heart rate percentage and is positive correlated to the plurality of speed;
S705: Estimating a future total exercise time based on a default displacement and the maximal oxygen consumption, wherein the future total exercise time is positive correlated to the default displacement, and wherein the future total exercise time is negative correlated to the maximal oxygen consumption;
S706: Receiving an environmental condition from the terminal device;
S707: Calibrating the future total exercise time by the environmental condition to generate an environment specific total exercise time;
S708: Generating a data array comprising the maximal oxygen consumption, the future total exercise time, and the environmental specific total exercise time;
S709: Sending the data array to the terminal device.

In one embodiment of the present invention, the order of step S702 and S703 are interchangeable.

In one embodiment of the present invention, the environmental condition can affect a person's exercise performance, wherein the environmental condition can be steepness, altitude, atmosphere pressure, wind speed, ambient temperature, etc. These environmental conditions can be obtained by various non-physiological sensors such as ambient temperature sensor, anemometer, GPS sensor, level sensor, atmosphere pressure sensor, etc. For example, a person's running speed can be greatly reduced while the person is running upwardly on a slope. It should be noticed that, the calibration of future total exercise time in step S707 can also be applied to the methods in Fig. 6, Fig. 7 and Fig. 9.

In one embodiment of the present invention, the heart rate percentage is calculated by taking the highest heart rate from the plurality of heart rate as denominator and each of the heart rate as numerator.

In one embodiment of the present invention, the heart rate percentage of a person while doing exercise should be around 60% or above. If any heart rate percentage calculated in step S702 is below 60%, the person may be considered not exercising, so the particular heart rate percentage below 60% may be omitted from the estimation of maximal oxygen consumption in step S704. It should be understood that the 60% was used as an example, the heart rate percentage of a person considered to be exercising may be different from one to another. Therefore, the heart rate percentage considering exercising may be customizable in the exercise monitoring device 100.

In one embodiment of the present invention, the data array can comprise the maximal oxygen consumption estimated in step S704, the future total exercise time estimated in S705, the environmental specific total exercise time calibrated in step S707. Furthermore, the data array can comprise at set of default displacements such as 3km, 5km, 10km, 21km, 42km, etc. And, each of the default displacement may be corresponding to a future total exercise time estimated in step S705 and an environmental specific total exercise time calibrated in step S707. In additional, the data array can also comprise a suggestion of warm up maximal oxygen consumption, wherein the suggested warm up maximal oxygen consumption can be used to determine whether warm up before exercise is enough or not. For example, a user can know the suggested warm up maximal oxygen consumption by the method in Fig. 10. And the user wearing the exercise monitoring device 100 can read the user's maximal oxygen consumption in real-time from the user interface 103 while doing warm up. Therefore, as the warm up going on, the user can know whether the user is ready for exercise by the real-time maximal oxygen consumption reaches the suggested warm up maximal oxygen consumption.

In one embodiment of the present invention, the terminal device can be the exercise monitoring device 100. Therefore, the heart rate is collected from the physiological sensor of the exercise monitoring device 100 and the displacement is collected from the non-physiological sensor of the exercise monitoring device 100. Alternatively, the terminal device can be any other monitoring device with both physiological sensor and non-physiological sensor. For example, a portable device with a heart rate sensor and a GPS sensor. Furthermore, the exercise monitoring device 100 can comprise an output socket (not shown), wherein the output socket enable the exercise monitoring device 100 to send the historical exercise data in step S701 or receive the data array in step S709 via a wire. Alternatively, the exercise monitoring device 100 can also comprise a wireless communication module (not shown), wherein the wireless communication module enables the exercise monitoring device 100 to send the historical exercise data in step S701 or receive the data array in step S709 wirelessly.

In another embodiment of the present invention, the terminal device can be a computing device with user input function, so the heart rate and the displacement are inputted by a user into the terminal device. Alternatively, the heart rate and the displacement can be transmitted from any other monitoring device into the computing device either wirelessly or with wire. The computing device can be such as personal computer, laptop, tablet pc, mobile device, etc.

In one embodiment of the present invention, various physiological parameters can be additional factors in the estimation of maximal oxygen consumption, such as heart rate variability, body temperature, body composition, blood glucose, blood pressure, etc. The body composition can comprise percentages of fat, bone, water and muscle in human bodies. Therefore, the estimation of maximal oxygen consumption can be personalized by the user of exercise monitoring device 100.

It should be noticed that, the aforementioned positive correlation or negative correlation cannot always be true when any other additional exercising factors comes into the estimation, for example a person with excellent running skill, which is an additional exercising factor, can increase displacement while maintaining the same maximal oxygen consumption.

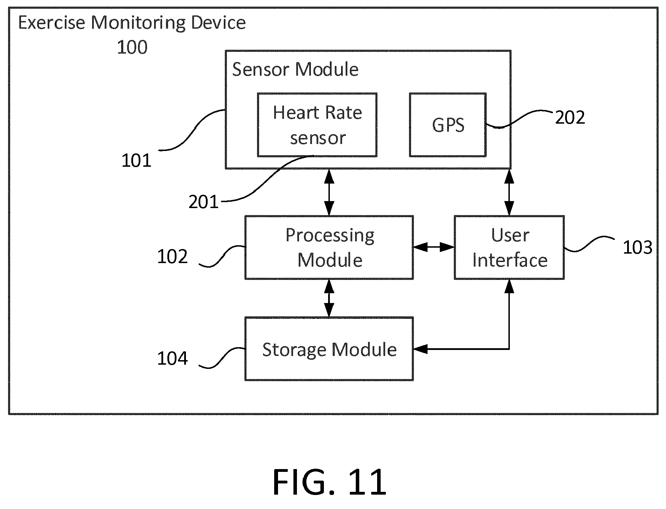

Fig. 11 is a schematic block diagram of the exercise monitoring device with a heart rate sensor and a GPS according to at least one embodiment of the present invention.

The exercise monitoring device 100 comprises a sensor module 101, a processing module 102, a user interface 103, and a storage module 104, wherein the sensor module 101 further comprises a heart rate sensor 201 and a GPS 202 in comparison to Fig. 1.

In one embodiment of the present invention, the heart rate sensor 201 can be used to record heart rate of a person and send it to the processing module 102 as physiological data. The GPS 202 can record a person's coordination in order to obtain the person's displacement and thus speed, and the GPS can send the speed as exercise data to the processing module. The processing module 102 can carry out at least one of the methods which is stored in the storage module 104 as shown previously, for example the method in Fig. 6.

In one embodiment of the present invention, the heart rate sensor 201 comprises at least two electrodes (not shown), wherein the at least two electrodes is electrically connected with the user's skin to detect the user's heart rate.

In one embodiment of the present invention, the exercise monitoring device 100 can further comprise an analog front-end (not shown), as known as AFE, wherein the heart rate sensor 201 can send analog signals to the processing module 102 via the analog front-end, and wherein the analog front-end can be for example but not limited to AD8232 from Analog Devices, ADS 1191 from Texas Instruments.

In one embodiment of the present invention, the heart rate sensor 201 comprises at least a light source (not shown), wherein the heart rate sensor 201 is an optical heart rate sensor that detects the user's heart rate.

In one embodiment of the present invention, the GPS 202 can be for example but not limited to SiRFstarV 5E from CSR, EVA-M8M from U-Blox.

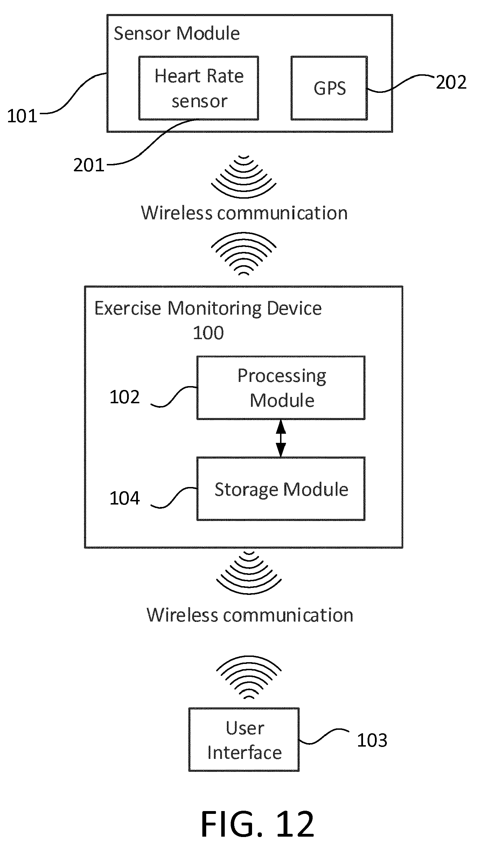

Fig. 12 is a schematic block diagram of the exercise monitoring device with 100 an external sensor module 101 and an external user interface 103 according to at least one embodiment of the present invention.

Referring to Fig. 12 according to one embodiment of the present invention, the exercise monitoring device 100 can only comprise the processing module 102 and the storage module 104. In this case, the sensor module 101 can be a wearable device which can communicate with the exercise monitoring device 100 wirelessly, therefore sending the physiological data (heart rate) and the exercise data (speed) to the processing module 102 via wireless communication. The same concept can be applied to the user interface 103 as well, wherein the user interface 103 can be realized by a mobile device or portable device. For example, user of the exercise monitoring device 100 can estimate the maximal oxygen consumption based on the heart rate and speed received wirelessly from the sensor module 101 and send the maximal oxygen consumption in real-time to a mobile phone of the user, wherein the mobile phone can display the maximal oxygen consumption.

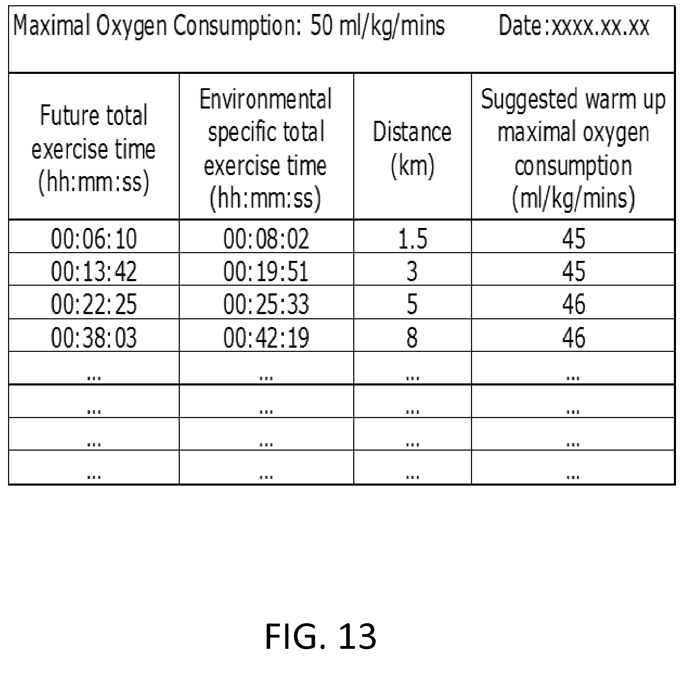

Fig. 13 is a schematic example of the data array generated in Fig. 10.

Referring to Fig. 13, the data array for example as shown in Fig. 13 comprising to the maximal oxygen consumption, the future total exercise time, the environmental specific total exercise time. Furthermore, the data array can comprise but not limited to a set of default displacements such as 3km, 5km, 10km, 21km, 42km, etc. And, each of the default displacement may be corresponding to a future total exercise time and an environmental specific total exercise time. In additional, the data array can also comprise a suggestion of warm up maximal oxygen consumption. Alternatively, the default displacement can be defined by the user of the exercise monitoring device 100 using the input unit of the user interface 103. It should be noticed that, the default displacement can be displacement of exercise such as a running distance, a climbing altitude, a cycling distance, etc.

Previous descriptions are only embodiments of the present invention and are not intended to limit the scope of the present invention. Many variations and modifications according to the claims and specification of the disclosure are still within the scope of the claimed invention. In addition, each of the embodiments and claims does not have to achieve all the advantages or characteristics disclosed. Moreover, the abstract and the title only serve to facilitate searching patent documents and are not intended in any way to limit the scope of the claimed invention.

## Claims

1. A maximal oxygen consumption estimation method, comprising:
receiving a physiological data from a first sensor;
estimating a rate of stamina consumption base on the physiological data by a processing module;
calculating an all-out exercise time base on the rate of stamina consumption by the processing module;
estimating an exercise capability base on the all-out exercise time by the processing module;
receiving an exercise data from a second sensor;
calculating a kinetic energy exertion base on the exercise data by the processing module;
estimating an average oxygen consumption base on the kinetic energy exertion by the processing module;
estimating a maximal oxygen consumption base on the average oxygen consumption and the exercise capability by the processing module;
sending the maximal oxygen consumption to a user interface;
displaying the maximal oxygen consumption by the user interface.

2. The maximal oxygen consumption estimation method according to claim 1, wherein the first sensor is a heart rate sensor, and the physiological data is heart rate.

3. The maximal oxygen consumption estimation method according to claim 1, wherein the first sensor comprises at least two electrodes.

4. The maximal oxygen consumption estimation method according to claim 1, wherein the first sensor comprises an optical heart rate sensor.

5. The maximal oxygen consumption estimation method according to claim 1, wherein the first sensor comprises an optical oxygen concentration sensor.

6. The maximal oxygen consumption estimation method according to claim 1, wherein the second sensor comprises a GPS sensor, and the exercise data is speed.

7. The maximal oxygen consumption estimation method according to claim 1, wherein the second sensor comprises a cycling power sensor.

8. The maximal oxygen consumption estimation method according to claim 1, wherein the second sensor comprises a motion sensor.

9. The maximal oxygen consumption estimation method according to claim 8, wherein the second sensor further comprises a gyroscope.

10. A future total exercise time estimation method, comprising:
receiving a physiological data from a first sensor;
estimating a rate of stamina consumption base on the physiological data by a processing module;
calculating an all-out exercise time base on the rate of stamina consumption by the processing module;
estimating an exercise capability base on the all-out exercise time by the processing module;
receiving an exercise data from a second sensor;
calculating a kinetic energy exertion base on the exercise data by the processing module;
estimating an average oxygen consumption base on the kinetic energy exertion by the processing module;
estimating a maximal oxygen consumption base on the average oxygen consumption and the exercise capability by the processing module;
estimating a future total exercise time base on the maximal oxygen consumption and a default displacement;
sending the future total exercise time to a user interface;
displaying the future total exercise time by the user interface.

11. The maximal oxygen consumption estimation method according to claim 10, wherein the first sensor is a heart rate sensor, and the physiological data is heart rate.

12. The maximal oxygen consumption estimation method according to claim 10, wherein the first sensor comprises at least two electrodes.

13. The maximal oxygen consumption estimation method according to claim 10, wherein the first sensor comprises an optical heart rate sensor.

14. The maximal oxygen consumption estimation method according to claim 10, wherein the first sensor comprises an optical oxygen concentration sensor.

15. The maximal oxygen consumption estimation method according to claim 10, wherein the second sensor comprises a GPS sensor, and the exercise data is speed.

16. The maximal oxygen consumption estimation method according to claim 10, wherein the second sensor comprises a cycling power sensor.

17. The maximal oxygen consumption estimation method according to claim 10, wherein the second sensor comprises a motion sensor.

18. The maximal oxygen consumption estimation method according to claim 17, wherein the second sensor further comprises a gyroscope.

19. A future total exercise time estimation method, comprising:
receiving a historical exercise model from a terminal device, wherein the historical exercise model comprises a plurality of heart rate and a plurality of displacement corresponding to the heart rate;
calculating a plurality of heart rate percentage based on the plurality of heart rate;
calculating a plurality of speed based on the plurality of displacement;
estimating a maximal oxygen consumption based on the plurality of heart rate percentage and the plurality of speed, wherein the maximal oxygen consumption is negative correlated to the plurality of heart rate percentage and positive correlated to the plurality of speed;
estimating a future total exercise time based on a default displacement and the maximal oxygen consumption, wherein the future total exercise time is positive correlated to the default displacement, and wherein the future total exercise time is negative correlated to the maximal oxygen consumption;
generating a data array comprising the maximal oxygen consumption and the future total exercise time;
sending the data array to the terminal device.

20. The future total exercise time estimation method according to claim 19, further comprising:
receiving an environmental condition from the terminal device;
calibrating the future total exercise time by the environmental condition to generate an environmental specific total exercise time;
wherein, the data array further comprising the environmental specific total exercise time.

21. The future total exercise time estimation method according to claim 20, wherein the environmental condition is obtained by a GPS.

22. The future total exercise time estimation method according to claim 20, wherein the environmental condition is ambient temperature.

23. The future total exercise time estimation method according to claim 19, wherein the heart rate is collected from a physiological sensor.

24. The future total exercise time estimation method according to claim 23, wherein the physiological sensor is a heart rate sensor comprising at least two electrodes.

25. The future total exercise time estimation method according to claim 23, wherein the physiological sensor is an optical heart rate sensor.

26. The future total exercise time estimation method according to claim 19, wherein the displacement is collected from a non-physiological sensor.

27. The future total exercise time estimation method according to claim 26, wherein the non-physiological sensor is a GPS.

28. The future total exercise time estimation method according to claim 26, wherein the non-physiological sensor is a motion sensor.

29. The future total exercise time estimation method according to claim 19, wherein receiving a historical exercise model from the terminal device by wireless communication.

30. The future total exercise time estimation method according to claim 19, wherein receiving a historical exercise model from the terminal device by a wire.
